# EUROPEAN PATENT APPLICATION

(11) **EP 3 964 204 A1**
(43) Date of publication of application: **09.03.2022**
(21) Application number: 20305993.6
(22) Date of filing: 08.09.2020
(51) Int. Cl.: A61K 31/137, A61K 31/27, A61K 31/4245, A61K 31/445, A61K 31/4468, A61K 31/495, A61K 31/496, A61K 31/506, A61K 31/513, A61K 31/5375, A61K 31/7105, A61K 45/06, A61P 9/00, A61P 9/10

(54) **LSD1 INHIBITORS FOR USE IN THE TREATMENT AND PREVENTION OF FIBROSIS OF TISSUES**

(71) Applicant: Université d'Aix-Marseille, 13007 Marseille (FR); Institut National de la Santé et de la Recherche Médicale, 75013 Paris (FR)
(72) Inventor: PUCEAT, Michel, 13014 Marseille (FR)
(74) Representative: LLR

(57) **Abstract**

An LSD1 inhibitor for use in preventing, reducing or stopping a development of a fibrosis of a myocardial tissue, said tissue having been submitted to an ischemic or a mechanical stress.

## Description

The present invention relates to the formation of fibrosis in various tissues of the body, and to compounds and compositions which can be used for alleviating or limiting the development of such fibrosis. It relates more particularly to the prevention or reduction of fibrosis appearing in tissues subjected to a mechanical stress or an ischemic stress, such as myocardial infarction.

The invention therefore concerns compounds and compositions for their use for preventing, reducing or stopping development of fibrosis in tissue of the body, in particular of human body. The invention concerns more particularly the prevention, reduction or stopping of fibrosis in cardiac tissue.

Fibrotic diseases cause annually more than 800,000 deaths worldwide, whereof the majority accounts for lung and cardiac fibrosis. A pathological remodeling of the extracellular matrix either due to ageing or as a result of an injury or disease leads to fibrotic area within the organ. In the heart, fibrosis as a scar or more diffuse cause several cardiac dysfunctions either by reducing the ejection fraction due to a stiffened myocardial matrix, or by impairing electric conductance, or they can even lead to death.

Cardiac fibrosis is a consequence of the death of myocytes during an attack or during acute or chronic suffering of the heart, including myocardial infarction.

Fibrosis has multiple origins but an important one is epithelial-mesenchymal transition (EMT) of endothelial, including endocardial and epicardial, cells of the heart, converting themselves and becoming fibroblasts and then myofibroblasts, secreting extracellular matrix (ECM). Although this mechanism is primarily protective, the overactivation of this process causes thickening and fibrosis of the cardiac tissue.

Cardiac fibrosis (scarring) is a common finding in many forms of heart failure. In the setting of a heart attack, scarring is typically localised to the region of heart damage whilst in other forms of heart failure the scarring is widespread.

Localised fibrosis is also found in cardiac tissue of people who had undergone a surgical repair to remedy congenital cardiac pathologies. When operated in the childhood, mechanical stress due to chronic volume and pressure overload most often of the right ventricle that develops over time and results in fibrosis of the myocardial tissue when becoming adult. Such fibrosis is present only in a limited defined area of the myocardial tissue of the ventricle.

Currently used therapeutics to impact the fibrotic response in injuries are angiotensin (AT)-converting enzyme and ATI receptor antagonist, β-blockers, endothelin antagonists, as well as statins. There are also approaches to influence fibroblast activation by blocking TGFβ or Smad3 signaling. Other therapeutic strategies include the application of growth factors and cytokines such as VEGF, IGF-1, HGF, NRG-1, EGF, FGF, and, TGFβ to induce cardiac repair (Hinderer, Advanced drug delivery review, 2019, 77-82). However, all these growth factors feature pleiotropic functions limiting their use as therapeutic targets.

However, there is still an important need of means for effectively preventing, stopping, controlling or reducing fibrosis of the tissue of the body, more particularly tissues which have been submitted to an ischemic or mechanical stress.

Surprisingly, it has now been found in the context of the present invention, that a class of compounds, LSD1 inhibitors, can fight against the development of fibrosis in such situations.

The present invention is therefore relating to the use of LSD1 inhibitors in the treatment of fibrosis in tissues of a subject in need thereof.

This is why the present invention relates to LSD1 inhibitor for use in preventing, reducing or stopping a development of a fibrosis of a tissue ; more particularly, for use in preventing, reducing or stopping a development of a fibrosis of a tissue having been submitted to an ischemic or a mechanical stress.

It is to be understood that the fibrosis may be following the ischemic or mechanical stress, or consecutive to said stress. However, it could be several days, weeks or even years between the ischemic or mechanical stress and the onset of fibrosis. Typically, the fibrosis is the consequence of an ischemic or mechanical stress event.

The use of LSD1 inhibitor according to the present invention is particularly intended for preventing, reducing or stopping the development of fibrosis in myocardial tissue., Such fibrosis is in particular a myocardial fibrosis resulting from an ischemic event, such as myocardial infarction or resulting from mechanical stress event, such as a heart surgery of an individual having a tetralogy of Fallot.

The invention is also relating to the use of an LSD1 inhibitor for the preparation of a pharmaceutical composition for use in the treatment of fibrosis in tissue of a subject, wherein said tissue has been submitted to an ischemic or mechanical stress, and to such compositions.

The invention is relating to compositions for use in the treatment of fibrosis of a tissue following ischemic or mechanical stress, containing at least an LSD1 inhibitor. The LSD1 inhibitor(s) can be the sole active ingredient, or other active ingredients can be present in the compositions.

The invention is also relating to a method for treatment of fibrosis in tissues of an subject, wherein said tissues have been submitted to an ischemic or mechanical stress, comprising the administration of at least an LSD1 inhibitor.

The LSD family belongs to the flavoenzyme-dependent demethylases. There are two members in this family: LSD1 (also called KDM1A) and LSD2 (also known as KDM1B). Crystallographic studies have shown that LSD1 and LSD2 share a conserved C-terminal amino oxidase domain (AOD) and a SWIRM domain. LSD1 functions to demethylate mono- and di-methylated lysines, in particular H3K4mel/2, H3K9mel/2, and non-histone proteins including p53, E2F1 and DNMTI. LSD1 is present in different protein complexes, such as the HDAC/CoREST/REST complex and the Mi- 2/nucleosome remodeling and deacetylase (NuRD) complex, and displays diverse functions.

As used herein, the term "LSD1" refers to a protein called lysine (K)-specific demethylase 1A (LSD1) or lysine-specific histone demethylase 1A (KDM1A) (UniProtKB/Swiss-Prot Number: 060341 in humans), that is also known as: AOF2, BHC110, KDM1, LSD1, CPRF, and lysine demethylase 1A. In humans, this enzyme is encoded by the KDM1A gene, which is situated on the short (p) arm of human chromosome 1 at position 36.12 (Gene ID: 23028). While LSD1 is involved in a wide variety of normal physiological processes, including stem cell maintenance and differentiation, it is also a key player in oncogenic processes, including compromised differentiation, enhanced cell motility and metabolic reprogramming. Therefore, the inhibition of LSD1 is considered as a potential therapeutic approach for the treatment for cancer, and a large number of LSD1 inhibitors have been reported and are currently undergoing clinical assessment for the treatment of acute myeloid leukemia, small-cell-- lung cancer, for instance.

Abdel Magid et al (ACS Med. Chem. Lett. 2017) is relating to 5-cyano indole derivatives which are LSD1 inhibitors and may be useful for treatment of B cell lymphoma, acute myeloid leukemia, various cancers such as gastric cancer, endometrial carcinoma and soft tissue carcinoma and sick cell disease.

Mc Donald et al (Nat. Struct. Mol. Biol., 2011) have studied genome-scale epigenetic reprogramming during epithelial to mesenchymal transition, and the possible role of LSD1 in this mechanism. Possible applications are DNA damage repair and involvement in cancer and chemioresistance.

Stratton et al (J. Mol. Cell Cardiol. 2016) are studying epigenetic regulation in cardiac fibrosis. Enzymes which are cited are histone deacetylases, histone acetyltransferases and lysine methyltransferases.

Wang et al (J. Mol. Cell Cardiol. 2018 is discussing the underlying mechanism responsible for the cardioprotective effects of riboflavin which could act in part through activation of LSD1 cellular activity at least in the H9C2 cell line. The authors suggest that activation of LSD1 may serve as a potential therapeutic target for cardiovascular disease treatment.

WO 2019/068326 is relating to the use of LSD1 inhibitors for the treatment and prevention of LMNA-related dilated cardiomyopathies. Dilated cardiomyopathy is an inherited cardiomyopathy characterized by an increase in both myocardial mass and volume, which compromises cardiac contractibility and ultimately results in reduced left ventricular function. As defined in WO 2019/068326, cardiomyopathy" refers to a group of diseases that adversely affect cardiac cell tissue leading to measurable deterioration in myocardial function (e.g., systolic function, diastolic function), and that are unexplained by abnormal loading conditions, congenital cardiac abnormalities, and ischemic heart disease.

WO 2012/156537 is relating to the use of an LSD1 inhibitor to reduce platelets and for the prevention of thrombosis and thrombus formation.

According to the present invention, LSD1 inhibitor can be used in the treatment of fibrosis in a tissue which has been previously submitted to an ischemic or mechanical stress.

The LSD1 inhibitor will be in a preferred embodiment, used in a subject in need thereof. Said subject is advantageously a mammal, and in particular a human being. the term "patient" can also be used in the present specification, to refer to the subject, in particular human being, treated with the LSD1 inhibitor according to the invention. The term individual may also be used.

In particular, the subject may be a human being who has been subjected to an ischemic stress such as myocardial infarction. The ischemic stress can have occurred for example between 2 or 3 months prior to the beginning of the use of LSD1 inhibitors according to the invention.

According to other embodiments, the subject may be a human being whose tissue have been subjected to a mechanical stress, such as the stress resulting from a heart surgery.

LSD1 inhibitors are used according to the invention in preventing or reducing the development of a fibrosis. By "reducing" is intended the use in limiting partly or completely the development of a fibrosis ; accordingly, in some embodiments, an LSD1 inhibitor will stop the development of a fibrosis at an early stage : the fibrosis of the tissue will therefore be lowered, even almost completely. In some embodiments, the use of an LSD1 inhibitor will prevent the formation of fibrosis in the treated tissue, where fibrosis will therefore be absent. Reducing is also comprising delaying or slowering the development of the fibrosis.

By preventing is intended the use before the onset of the fibrosis, so that the fibrosis will not appear, or will be lowered and/or delayed.

Except if otherwise stated, the expression "an LSD1 inhibitor" is intended to mean "at least one LSD1 inhibitor". Accordingly, it encompasses either the use of only one LSD1 inhibitor or of mixtures of two or more LSD1 inhibitors.

An LSD1 inhibitor can be used according to the present invention in a composition containing it. Said composition will contain LSD1 inhibitor and at least a physiologically acceptable medium. In particular, the composition can be a pharmaceutical composition.

According to an embodiment of the invention, an LSD1 inhibitor is used in preventing, reducing or stopping a development of a fibrosis of the myocardial tissue further to a myocardial infarction.

Myocardial infarction occurs when blood flow to the heart muscle is abruptly cut off, causing tissue damage. This is usually the result of a blockage in one or more of the coronary arteries. If impaired blood flow to the heart lasts long enough, it triggers a process called the ischemic cascade; the heart cells in the territory of the blocked coronary artery die (infarction), chiefly through necrosis, and are not replaced by functional living cells. A collagen scar forms in their place. When an artery is blocked, cells lack oxygen, needed to produce ATP in mitochondria. ATP is required for the maintenance of electrolyte balance, particularly through the Na/K ATPase. This leads to an ischemic cascade of intracellular changes, necrosis and apoptosis of affected cells.

Cells in the area with the worst blood supply, just below the inner surface of the heart (endocardium), are most susceptible to damage. Ischemia first affects this region, the subendocardial region, and tissue begins to die within 15-30 minutes of loss of blood supply. The dead tissue is surrounded by a zone of potentially reversible ischemia that progresses to become a full-thickness transmural infarct.

Tissue death and myocardial scarring alter the normal conduction pathways of the heart, and weaken affected areas.

The ischemic stress of the tissue is inducing fibrosis.

Fibrosis will therefore appear in a limited area of the myocardial tissue of the left ventricle, downstream the obstruction of the coronary artery.

In other embodiments the invention is relating to an LSD1 inhibitor for its use in preventing, reducing, or stopping a development of a fibrosis of myocardial tissue of the right ventricle following a mechanical or ischemic stress. In particular it relates for use in preventing, reducing or stopping a development of a fibrosis of myocardial tissue of the right ventricle of the heart, following pressure and/or volume overload of the right ventricle. This condition appears for instance in congenital heart disease.

Due to improved surgical management of congenital heart disease (CHD) in infancy and childhood, the number of adult patients with CHD has increased rapidly. Current prevalence among adults is about 3 per 1.000 persons, 2 5% with severe lesions and 36% with moderate lesions. This includes a range of defects affecting the right ventricle (RV). In CHD, the RV is often affected by a combination of anatomical factors and acquired factors, such as residual lesions after surgery. This is in contrast to acquired heart disease, which predominantly affects the left ventricle (Woudstra et al. Cardiovascular Research, 2017).

The invention is therefore relating to an LSD1 inhibitor for use in preventing, reducing or stopping a development of a fibrosis of a tissue further to surgery of an individual having a congenital heart disease, and in particular of an individual having a condition selected from tetralogy of Fallot, double outlet right ventricle, transposition of great arteries and Systemic right ventricle.

In all these conditions, post-repair of the cardiac congenital malformation, there is a localized fibrosis, associated to the pressure and volume overload of the RV.

The invention is relating to an LSD1 inhibitor for use in preventing, reducing or stopping and in particular in inhibiting or controlling myocardial localized fibrosis.

Localized fibrosis should be understood as a fibrosis present only in a part of the myocardial tissue, in particular only in a part of a ventricle, as opposed to fibrosis widespread in most of the myocardial tissue.

LSD1 inhibitors useful for the present invention can be reversible inhibitors or irreversible inhibitors.

As used herein, the term "LSD1 inhibitor" includes and encompasses any active agent or compound that, irrespective of its mechanism(s) of action, reduces or inhibits the accumulation, function or stability of the histone demethylase LSD1, or that decreases or prevents expression of the KDM1A gene. The terms "reduce", "inhibit", "decrease" and "prevent" are used relative to a control. One skilled in the art would readily identify the appropriate control to be used for comparison. For example, reduction or inhibition of the accumulation, function or stability of LSD1 in a subject or cell that is treated with a LSD1 inhibitor is assessed by comparison to the accumulation, function or stability of LSD1 in a subject or cell that is not treated with the LSD1 inhibitor. Similarly, decrease or prevention of the expression of the KDM1A gene in a subject or cell that is treated with a LSD1 inhibitor is assessed by comparison to the decrease or prevention of the expression of the KDM1A gene in a subject or cell that is not treated with the LSD1 inhibitor. Assays and methods to assess LSD1 inhibition are known in the art (see, for example, Hayward and Cole, Methods Enzymol., 2016, 573: 261-278). LSD1 inhibitors that are suitable for use in the present invention may be reversible or irreversible, direct or indirect.

Suitable LSD1 inhibitors may be nucleic acids, peptides, polypeptides, peptidomimetics, carbohydrates, polysaccharides, lipopolysaccharides, lipids, and other organic or inorganic molecules. In the context of the present invention, a LSD1 inhibitor may be any member of a class of compounds (e.g., a small molecule, or an antibody or fragment or derivative of such antibody) that binds LSD1 and inhibits a biological activity (e.g., demethylase activity) of LSD1 or a protein complex in which LSD1 exerts its function (e.g. , LSD1 complexed to co-REST and/or other protein members of the nucleosome). A LSD1 inhibitor may also be any member of a class of compounds that decreases the expression of a nucleic acid encoding a LSD1 protein (e.g. , a nucleic acid that inhibits the transcription or translation of the KDM1A gene or that mediates RNA interference, RNAi).

### A. Small Molecule Inhibitors of LSD1

As used herein, the term "small molecule" refers to a molecule having a molecular weight of less than 1,000 daltons, preferably of less than 700 daltons.

Since LSD1 and the monoamine oxidases, MAO-A and MAO-B, which play key roles in the clearance of neurotransmitters, share a common mechanism for the oxidative cleavage of the unactivated nitrogen-carbon bonds of their substrates, many of the known MAO inactivators have been tested as LSD1 inhibitors (Lee et ah , Chem. Biol., 2006, 13(6): 563-567). Thus, in certain embodiments of the present invention, the LSD1 inhibitor is a selective LSD1 inhibitor, while in other embodiments of the present invention, the LSD1 inhibitor is dual LSD1/MAO inhibitor.

As used herein, the term "selective LSD1 inhibitor" refers to an LSD1 inhibitor, which has a half maximal inhibitory concentration (i.e., IC₅₀) value for LSD1 that at least 2-fold lower than its IC₅₀ values for other FAS utilizing enzymes (i.e., LSD2, MAO- A and MAO-B). Preferably, a selective LSD1 inhibitor has an IC₅₀ value for LSD1 which is at least 5-fold, at least 10-fold, or at least 20-fold, lower than its IC₅₀ values for LSD2, MAO-A and MAO-B. More preferably, a selective LSD1 inhibitor has an IC₅₀ value that is at least 50-fold, or at least 100-fold, lower than its IC₅₀ values for LSD2, MAO- A and MAO-B. Even more preferably, a selective LSD1 inhibitor has an IC₅₀ value that is more than 100-fold lower than its IC₅₀ values for LSD2, MOA-A and MOA-B. One skilled in the art knows how to determine IC₅₀ values.

The terms "dual LSD1/MAO-A(B) inhibitor" "selective inhibitor of LSD1 and MAO-A(B)", "LSD1/MAO-A(B) inhibitor" and "dual LSD1/MAO-A(B) selective inhibitor" are used herein interchangeably. They refer to an LSD1 inhibitor which has IC₅₀ values for LSD1 and MAO- A (or MAO-B) that are at least 2-fold lower than its IC₅₀ value for MAO-B (or MAO-A, respectively). Preferably, they refer to an LSD1 inhibitor which has IC₅₀ values for LSD1 and MAO-A (or MAO-B) that are at least 5- fold, at least 10-fold, or at least 20-fold, lower than its IC₅₀ value for MAO-B (or MAO-A, respectively). More preferably, they refer to an LSD1 inhibitor which has IC₅₀ values for LSD1 and MAO-A (or MAO-B) that are at least 50-fold, or at least 100-fold, lower than its IC₅₀ value for MAO-B (or MAO-A, respectively). Even more preferably, they refer to an LSD1 inhibitor which has IC₅₀ values for LSD1 and MAO-A (or MAO- B) that are at more than 100-fold lower than its IC₅₀ value for MAO-B (or MAO-A, respectively).

**Irreversible Small Molecule LDS1 Inhibitors.** In certain embodiments, the LSD1 inhibitor is an irreversible small molecule LSD1 inhibitor.

Examples of irreversible small molecule inhibitors include, without limitation, pargyline (propargylamine) derivatives or analogs (Culhane et al, J. Am. Chem. Soc, 2006, 128: 4536-4537; Schmitt et al, J. Med. Chem., 2013, 56: 7334-7342), phenelzine derivatives or analogs (Prusevich et al, ACS Chem. Biol., 2014, 9: 1284-1293), and cyclopropylamine derivatives or analogs. Preferably, the LSD1 inhibitor is selected among cyclopropylamine derivatives or analogs, in particular among tranylcypromine (+/-) trans-2-phenylcyclopropylamine) derivatives.

Examples of tranylcypromine derivatives that can be used as LSD1 inhibitors in the practice of the present invention include, without limitation, the tranylcypromine derivatives developed by Oryzon Genomics (Barcelona, Spain), such as the ones described in WO 2010/043721, WO 2010/084160, WO 2011/042217, WO 2010/043721, WO 2011/035941, WO 2011/042217, WO 2011/131697, WO 2012/013727, WO 2012/013728, WO 2012/045883; WO 2013/057322, and WO 2013/057320; the tranylcypromine derivatives developed by GlaxoSmithKline, such as the ones described in WO 2012/135113; and the tranylcypromine derivatives developed by Takeda, such as the ones described in WO 2013/022047; and the cyclopropylamine derivatives developed by Incyte Corporation, such as the ones described in WO 2015/123437, WO 2015/123408, WO 2015/123424, and WO 2015/123424.

Other cyclopropylamine and arylcyclopropylamine derivatives and analogues that can be used as LSD1 inhibitors in the practice of the present invention include, without limitation, the ones described in US 2010/0324147; WO 2011/131576; Gooden et al, Bioorg. Med. Chem. Let., 2008 18: 3047-3051; 6827-6833; Ueda et al, J. Am. Chem. Soc, 2009, 131: 17536-17537; Mimasu et al, Biochemistry, 2010, 49: 6494-6503; Binda et al, J. Am. Chem. Soc, 2010, 132: 6827-6833; Benelkebir et al, Bioorg. Med. Chem., 2011, 19: 3709-3716; Valente et al, ACS Med. Chem. Lett., 2014, 6: 173-177; Khan et al, Med. Res. Rev., 2013, 33: 873-910; Vianello etal, Eur. J. Med. Chem., 2014, 86: 352-363; Valente et al, Eur. J. Med. Chem., 2015, 94: 163-174; Malcomson et al, FEBS J., 2015, 282: 3190-3198; Borrello et al, Bioorg. Med. Chem. Lett., 2017, 27: 2099-2101; and Trifiro et al, Future Med. Chem., 2017, 9: 1161-1174.

Several N-alkylated tranylcypromine derivatives, including ORY-1001 (Oryzon Genomics, Roche), ORY-2001 (Oryzon Genomics), ORY-3001 (Oryzon Genomics), GSK-2879552 (GlaxoSmithKline), INCB-059872 (Incyte), IMG-7289 (Imago Biosciences), and CC-90011 (Celgene), are currently in clinical trials for treatments of cancers, such as acute myeloid leukemia (AML), small cell lung cancer (SCLC), myelodysplasia syndrome (MDS), and non-Hodgkin's lymphoma (NHL), and for treatments of neurodegenerative diseases such as Alzheimer's disease, Parkinson's disease, Huntington's disease. Thus, in particular, in certain embodiments, the LSD1 inhibitor to be used in the practice of the present invention is selected from ORY-1001 (N1-[(1R,2S)-2-phenylcyclopropyl]-1,4-cyclohexanediamine, dihydrochloride, CAS Number 1431326-61-2, ORY-2001 (CAS No. : 1357362-02-7), ORY-3001, GSK-2879552 (CAS No.1401966-69-5), INCB-059872, IMG-7289 (CAS number: 1990504-34-1), and CC-90011 (CAS NO: 2097523-60-7).

Other specific tranylcypromine derivatives that can be used as LSD1 inhibitors to be used in the practice of the present invention include, for example, RN-1 and RN-7 (Neelamegam et al, ACS Chem. Neurosci., 2012, 3: 120-128; Cui et al, Blood, 2015, 126: 386-396; McGrath et al, Cancer Res., 2016, 76: 1975-1988), GSK-2699537 (developed by GlaxoSmithKline) and T-3775440 (developed by Takeda; Ishikawa et al, Mol. Cancer Ther., 2017, 16(2): 273-284).

In a particular embodiment of the present invention, the selective LSD1 inhibitor is GSK-LSD1 (N-[(1R,2S)-2-phenylcyclopropyl]piperidin-4-amine dihydrochloride; Cas Number: 1431368-48-7 (for the free base)). GSK-LSD1, is a selective and irreversible inhibitor of LSD1:
GSK-LSD1 inhibits LSD1 with an IC₅₀ of 16 nM and is more than 1000 fold selective over other closely related FAD utilizing enzymes {i.e., LSD2, MAO-A, MAO-B).

In another particular embodiment of the present invention, the selective LSD1 inhibitor is GSK-2879552 (4-[[4-[[((1R,2S)-2-Phenylcyclopropyl)amino]-methyl] piperidin-1-yl] methyl] benzoic acid; Cas Number: Cas 1401966-69-5). GSK- 2879552, is a selective and irreversible inhibitor of LSD1.

In yet another particular embodiment of the present invention, the selective LSD1 inhibitor is ORY-1001 (rel-N¹-[(1R,2S)-2-phenylcyclopropyl]-1,4-cyclohexanediamine, dihydrochloride; CAS Number: 1431303-72-8 for the 2 HCI salt; CAS Number: 1431303-71-7 for the xHCl salt; and CAS Number: 1431304-21-0 for the free base). ORY-1001 is a selective inhibitor of LSD1 (IC₅₀ < 20 nM),

Another example of LSD1 inhibitor is 4SC-202 (4SC AG).

**Reversible small molecules inhibitor.** In certain embodiments, the LSD1 inhibitor is a reversible small molecule LSD1 inhibitor.

Examples of reversible small molecule LSD1 inhibitors include, without limitation, LSD1 inhibitors containing a benzohydrazide scaffold, such as the ones described in Soma et al, J. Med.Chem., 2013, 56: 9496-9508; Zhou et al, Bioorg. Med. Chem. Lett, 2016, 26: 4552-4557; WO 2013/025805, WO 2014/205213; WO 2017/004519), for example HCI-2509 (also named SP2059 or LSD1-C12 - CAS Number: 1423715-09-6) (Sankar et al, Clin. Cancer Res., 2014, 20: 4584-4597; Kiskus et al, Leukemia, 2014, 28: 2155-2164) or GSK-354 (Dhanak, Proceedings of the 104th Annual Meeting of the American Association for Cancer Research, 2013, Drugging the cancer genome).

Other examples of reversible LSD1 inhibitors suitable for use in the context of the present invention include dithiocarbamates-based compounds, such as the ones described by Zheng et al, J. Med. Chem., 2013, 56: 8543-8560; Ye et al, Med. Chem. Commun., 2014, 5: 650-654.

Yet other examples of reversible LSD1 inhibitors suitable for use in the present invention include pyridine, pyrazine and pyrimidine derivatives and analogs, such as the ones developed by Incyte Corporation and described in WO 2016/007722, WO 2016/007727, WO 2016/007731 and WO 2016/007736; and the ones described by Li et al, ACS Med. Chem. Lett., 2017; 8: 384-389 and Wang et al, Eur. J. Med. Chem., 2017, 125: 940-951.

Still other examples of reversible LSD1 inhibitors suitable for use in the present invention include diverse triazole- and tetrazole-based compounds, such as the ones described by Kutz et al, Med. Chem. Commun., 2014, 5: 1863-1870; WO 2015/120281, and the reversible LSD1 inhibitors with a 5-hydroxypyrazole scaffold, such as the ones described by Mould et al, Bioorg. Med. Chem. Lett., 2017, 27: 3190-3195.

Other examples of reversible LSD1 inhibitors include polyamine analogs such as bisguanidine and biguanide (Huang et al, PNAS, 2007, 104: 8023-8028; Zhu et al, Amino Acids, 2012, 42, 42: 887-898); bisurea and bisthiourea derivatives (WO 2007/021839; WO 2008/127734; WO 2011/022489; Sharma et al, J. Med. Chem., 2010, 43: 5189-5212; Nowotarski et al, Bioorg. Med. Chem., 2015, 23: 1601-1612); guanidinium-based compounds (Wang et al, Cancer Res., 2011, 71: 7238-7249), such as CBB1007; lysine-based compounds (WO 2014/084298); and oligoamine analogues {i.e., long chain polyamine analogues) (Huang et al, Clin. Cancer Res., 2009, 15: 7217- 7228).

The chromenone namoline and namoline analogs have also been described as a reversible LSD1 inhibitors (Willmann et al, Int. J. Cancer, 2012, 131: 2704-2709; Schulz-Fincke et al., Mol. Cancer Ther., 2015 14(12 Suppl 2): Abstract nr B81). Other examples of reversible small molecule LSD1 inhibitors include the acyclic ditherpenoid geranylgeranoic acid and its derivatives (Sakane et al, Biochem. Biophys. Res. Commun., 2014, 444: 24-29).

Linear and cyclic peptides have also been developed as reversible small molecule LSD1 inhibitors. Examples of such peptides include, without limitation, the ones described in Kumarasinghe et al, ACS Med. Chem. Lett., 2013, 5: 29-33; Tortorici et al, ACS Chem. Biol., 2013, 8: 1677-1682; Kakizawa et al, Bioorg. Med. - Chem. Lett., 2015, 25: 1925-1928; and Amano et al, Bioorg. Med. Chem., 2017, 25: 2617-2624.

As will be recognized one skilled in the art, any reversible small molecule LSD1 inhibitor may find application in the present invention, including the reversible small molecule LSD1 inhibitors currently in development, including, without limitation, the carboxamides derivatives (Sartori et al., J. Med. Chem., 2017, 61: 1673-1692; Vianello et al., J. Med. Chem. 2017, 60: 1693-1715), benzesulfonamide derivatives (Xi et al., Bioorg. Chem., 2017, 72: 182-189), etc...

### B. LSD1 RNA Interfering Agents

Other agents that can be used as LSD1 inhibitors include RNA interfering agents.

As used herein, the term "RNA interference" (or "RNAi") has its art understood meaning and refers to a biological process in which RNA molecules silence, inhibit or down-regulate gene expression by causing the destruction, degradation and/or cleavage of specific mRNA molecules or by blocking the translation thereof. As known in the art, RNA interference is now exploited in therapy. Indeed, RNAi can be initiated by the hand of man, for example, to silence the expression of target genes. The terms "RNAi agent" and "RNA interfering agent" are used herein interchangeably. They refer to any RNA molecule that is capable of specifically inhibiting or down-regulating the expression of a target gene (here the KDM1A gene). By "silencing, inhibiting or down- regulating expression of a target gene", it is meant that the expression of the target gene, or level of RNA molecules or equivalent RNA molecules encoding one or more proteins or protein subunits, or activity of one or more proteins or protein subunits, is reduced below that observed in the absence of the RNAi agent.

In some embodiments of the present invention, an RNA interfering agent reduces the expression of the KDM1A gene or transcript to less than about 90%, 80%, 70%, 60%, 50%, 25%, 10%, 5%, 1%, or to less than 0.1% (for example less than about 10⁻⁴, 10⁻⁵, 10⁻⁶, 10⁻⁷, 10⁻⁸, 10⁻⁹, 10⁻¹⁰, 10⁻¹¹, 10⁻¹², 10⁻¹³, 10⁻¹⁴, or 10⁻¹⁵) of the expression of the KDM1A gene or transcript in the absence of the RNA interfering agent.

An RNAi agent may be any single-stranded RNA (e.g., mature miRNA, ssRNAi oligonucleotides, ssDNAi oligonucleotides) or double-stranded RNA (i.e., duplex RNA such as siRNA, Dicer-substrate dsRNA, shRNA, aiRNA, or pre-miRNA) that is capable of reducing or inhibiting the expression of a target gene or sequence (e.g., by mediating the degradation or inhibiting the translation of mRNAs which are complementary to the interfering RNA sequence) when the RNAi agent is in the same cell as the target gene or sequence. The term "RNAi agent" thus refers to the single- stranded RNA that is complementary to a target mRNA sequence (or circRNA sequence) or to the double-stranded RNA formed by two complementary strands or by a single, self- complementary strand. An RNAi agent may have substantial or complete identity to the target gene mRNA (or circRNA) or sequence, or may comprise a region of mismatch (i.e., a mismatch motif). Consequently, the term "RNAi agent" refers to a RNA molecule comprising a strand having a sequence sufficiently complementary to a target mRNA (or circRNA) sequence to direct target- specific RNA interference (RNAi) thereby inhibiting or down-regulating the expression of the target gene.

An RNAi agent can comprise naturally occurring RNA, synthetic RNA, or recombinantly produced RNA, as well as altered RNA that differs from naturally-occurring RNA by the addition, deletion, substitution and/or alteration of one or more nucleotides. Such alterations can include addition of non-nucleotide material, such as to the end of the molecule or to one or more internal nucleotides of the RNAi, including modifications that make the RNAi agent resistant to nuclease digestion. An RNAi agent may be administered in free (naked) form or by the use of delivery systems that enhance stability and/or targeting, or may be administered in the form of its precursor or encoding DNA.

In certain preferred embodiments of the present invention, an RNAi agent is a siRNA (small interfering RNA), a shRNA (short hairpin RNA), a micro-RNA (micro RNA), or an aiRNA (asymmetric interfering RNA). The development of any type of RNAi agent capable of specifically silencing, inhibiting or down-regulating the expression of a given target gene (here the TRPV2 gene) is within the capabilities of one skilled in the art. The use of RNAi agents, such as siRNAs, shRNAs, micro-RNAs or aiRNAs, to inhibit gene expression is well known in the art.

Preferably, the LSD1 RNA interfering agent used in the practice of the present invention is a small interfering RNA. The terms "small interfering RNA" and "siRNA" are used herein interchangeably. They refer to an RNA (or RNA analog) comprising between about 10-50 nucleotides (or nucleotide analogs) which is capable of directing or mediating RNA interference. Preferably, a siRNA comprises between about 15-30 nucleotides or nucleotide analogs, more preferably between about 16-25 nucleotides (or nucleotide analogs), even more preferably between about 18-23 nucleotides (or nucleotide analogs), and even more preferably between about 19-22 nucleotides (or nucleotide analogs) (e.g., 19, 20, 21 or 22 nucleotides or nucleotide analogs).

Starting from the sequence of the KDM1A gene or transcript to be silenced, one skilled in the art knows how to develop suitable siRNA molecules. Representative KDM1A transcripts include nucleotide sequences corresponding to any one the following sequences: (1) human LSD1 nucleotide sequences as set forth for example in GenBank Accession Nos. NM_015013.3, NP_001009999.1, and NM_001009999.2; (2) nucleotide sequences that share at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99% sequence identity with any one of the sequences referred to in (1); (3) nucleotide sequences that hybridize under at least low, medium or high stringency conditions to the sequences referred to in (1); (4) nucleotide sequences that encode any one of the following amino acid sequences: human LSD1 amino acid sequences as set forth for example in GenPept Accession Nos. NP_055828.2, NP_001009999.1 and 060341.2; (5) nucleotide sequences that encode an amino acid sequence that shares at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99% sequence similarity with any one of the sequences referred to in (4); and nucleotide sequences that encode an amino acid sequence that shares at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99% sequence identity with any one of the sequences referred to in (4).

siRNAs molecules may be prepared by any suitable method known in the art (see for example WO 02/44321). Sequence specific gene silencing can be achieved in mammalian cells using synthetic, short double- stranded RNAs that mimic the siRNAs produced by the enzyme dicer. siRNAs can be chemically or in viiro-synthesized or can be the result of short double- stranded hairpin-like RNAs (shRNAs) that are processed into siRNAs inside the cell. Synthetic siRNAs are generally designed using algorithms and a conventional DNA/RNA synthesizer. Suppliers include Ambion (Austin, TX, USA), ChemGenes (Ashland, MA, USA), Dharmacon (Lafayette, CO, USA), Glen Research (Sterling, VA, USA), MWB Biotech (Esbersberg, Germany), Proligo (Boulder, CO.), and Qiagen (Vento, The Netherlands). siRNAs can also be synthesized in vitro using kits such as Ambion's SILENCER^{™} siRNA Construction Kit.

Examples of LSD1 siRNA molecules have been described, for example, in Shi et al, Cell, 2004, 119: 941-953; Metzger et al, Nature, 2005, 437: 436-439; Zhu et al., PLoS ONE, 2008, 3: el446; Wang et al., Cell, 2009, 138: 660-672; Sun et al., Mol. Cell Biol, 2010, 30: 1997-2005; Hino et al, Nature Comm. 2012, 3: article number 758; and Xu et al., Cell Physiol. Biochem., 2013, 31: 854-862; and are commercially available from Santa Cruz Biotechnology, Inc. (Santa Cruz, CA, USA) and OriGene Technologies, Inc. (Rockville, MD, USA).

In particular, LSD1 inhibitors for use according to the invention are irreversible inhibitors, preferably irreversible selective inhibitors. Inhibitors particularly adapted are selected from tranylcypromine derivatives, and preferably phenylcyclopropyl piperidine derivatives. Such compounds encompass GSK-LSD1, of the following formula :

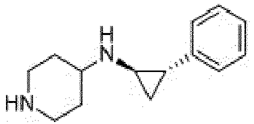

Others LSD1 inhibitors useful for the invention are substituted benzohydrazide analogs, disclosed for instance in WO 2017/004519. A reversible LSD1-inhibitor of interest is SP-2577, also named seclidemstat (CAS N° 1423715-37-0) of the following formula :

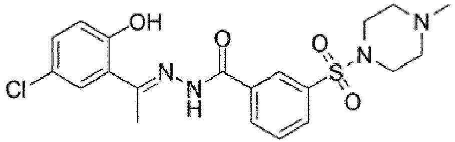

Others LSD1 inhibitors useful for the invention include SP2509 (CAS N° : 1423715-09-6) of the following formula :

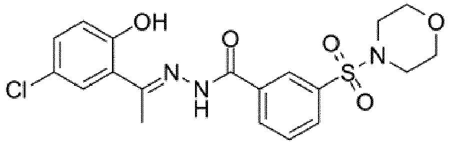

Advantageously, an LSD1 inhibitor for use according to the invention is selected from GSK-LSD1, GSK-2879552, ORY-1001, SP2509, SP2577, TCP, IMG-7289, INCB059872, ORY-2001, CBB3001, RG6016, INCB059872, IMG-7289 , CC-90011 and micro RNA .

According to one embodiment of the invention, an LSD1 inhibitor is used as the sole active ingredient.

According to another embodiment, an LSD1 inhibitor is used in association or combination with another ingredient, i.e. an active ingredient belonging to another pharmacological class.

In particular, the LSD1 inhibitor is used in association with at least another active ingredient selected from the group comprising beta blocking agents, anticoagulant, ACE (angiotensin-converting enzyme) inhibitors, Angiotensin II receptor blockers, Calcium channel blockers, diuretics, β-adrenergic receptors blockers, pacemaker channels modulators.

As mentioned above, an LSD1 inhibitor may be used in a composition containing said inhibitor, in particular a pharmaceutical composition. The LSD1 inhibitor is administered to a subject in need thereof through routes adapted by the man skilled in the art.

Various delivery systems are known and can be used to administer an LSD1 inhibitor of the present invention, including tablets, capsules, injectable solutions, encapsulation in liposomes, microparticles, microcapsules, etc. Methods of administration include, but are not limited to, dermal, intradermal, intramuscular, intraperitoneal, intralesional, intravenous, subcutaneous, intranasal, pulmonary, epidural, ocular, and oral routes. An LSD1 inhibitor of the present invention, or a pharmaceutical composition thereof, may be administered by any convenient or other appropriate route, for example, by infusion or bolus injection, by adsorption through epithelial or mucocutaneous linings (e.g. , oral, mucosa, rectal and intestinal mucosa, etc). Administration can be systemic or local. Parenteral administration may be directed to a given tissue of the patient, such as by catheterization. As will be appreciated by those of ordinary skill in the art, in embodiments where an LSD1 inhibitor is administered along with an additional therapeutic agent, the LSD1 inhibitor and the therapeutic agent may be administered by the same route (e.g. , orally) or by different routes (e.g., orally and intravenously).

LSD1 inhibitor can be administered in a composition comprising one or more pharmaceutically acceptable diluents or carriers.

A "pharmaceutical composition" is defined herein as comprising an effective amount of at least one LSD1 inhibitor of the invention, or a physiologically tolerable salt thereof, and at least one pharmaceutically acceptable carrier or excipient.

The term "physiologically tolerable salt" refers to any acid addition or base addition salt that retains the biological activity and properties of the free base or free acid, respectively, and that is not biologically or otherwise undesirable. Acid addition salts are formed with inorganic acids (e.g., hydrochloric, hydrobromic, sulfuric, nitric, phosphoric acids, and the like); or organic acids (e.g., acetic, propionic, pyruvic, maleic, malonic, succinic, fumaric, tartaric, citric, benzoic, mandelic, methanesulfonic, ethanesulfonic, p-toluenesulfonic, salicylic acids, and the like). Base addition salts can be formed with inorganic bases (e.g. , sodium, potassium, lithium, ammonium, calcium, magnesium, zinc, aluminum salts, and the like) or organic bases (e.g., salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethyl-aminoethanol, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins, and the like).

As used herein, the term "effective amount" refers to any amount of a molecule, compound, agent, or composition that is sufficient to fulfil its intended purpose(s), e.g., a desired biological or medicinal response in a cell, tissue, system or subject, such as, for example, the effect for which it is administered, to treat or prevent a disease, reduce an enzymatic activity, reduce one or more symptoms of the disease or condition, etc.). An example of an "effective amount" is a "therapeutically effective amount", which is an amount sufficient to contribute to the treatment or reduction of a symptom or symptoms of a disease. A "reduction" of a symptoms or symptoms means decreasing of the severity or frequency of the symptom(s), or elimination of the symptom(s). Another example of an "effective amount" is a "prophylactic effective amount", which is an amount sufficient to prevent or delay the onset (or reoccurrence) of a disease, pathology or condition, or reducing the likelihood of the onset (or reoccurrence) of the disease, pathology or condition, or their symptoms. The full therapeutic or prophylactic effect does not necessarily occur by administration of one dose, and may occur only after administration of a series of doses. Thus, a therapeutic or prophylactic effective amount may be administered in one or more administrations. The exact amounts will depend on the purpose of the treatment, and will be ascertainable by one skilled in the art using known techniques

The term "pharmaceutically acceptable carrier or excipient" refers to a carrier medium which does not interfere with the effectiveness of the biological and/or therapeutic activity of the active ingredient(s) and which is not excessively toxic to the host at the concentration at which it is administered. The term includes solvents, dispersion media, coatings, antibacterial and antifungal agents, isotonic agents, and adsorption delaying agents, and the like. The use of such media and agents for pharmaceutically active substances is well known in the art).

The terms "approximately" and "about", as used herein in reference to a number, generally include numbers that fall within a range of 10%, in particular 5%, in either direction of the number (greater than or less than the number) unless otherwise stated or otherwise evident from the context (except where such number would exceed 100% of a possible value).

According to an embodiment of the invention, an LSD1 inhibitor is used in preventing, reducing or stopping a development of a fibrosis of a tissue, wherein the tissue has been subjected to an ischemic stress which is myocardial infarction, and administration of the LSD1 inhibitor begins 2 or 3 months after said ischemic stress.

The treatment is continued during at least one month, preferably at least 6 months, and generally during at least one year.

In another embodiment, the invention is relating to an LSD1 inhibitor for use in preventing, reducing or stopping a development of a fibrosis of a tissue, wherein the tissue has been subjected to a mechanical stress resulting from a surgical operation and administration of the LSD1 inhibitor begins one week after said mechanical stress.

The beginning of the treatment will be adapted according to the monitoring of the onset of myocardial fibrosis, but is generally between one week and 10 years following said surgery, for instance between 1 months and 5 years after said surgery.

The invention is also relating to a product containing (i) at least an LSD1 inhibitor and (ii) an active ingredient selected from the group comprising beta blocking agents, anticoagulant, ACE (angiotensin-converting enzyme) inhibitors, Angiotensin II receptor blockers, Calcium channel blockers, diuretics, β-adrenergic receptors blockers, pacemaker channels modulators and digoxin (digitalis) as a combined preparation for simultaneous, separate or sequential use in preventing or reducing a development of a fibrosis of a tissue, said tissue having been submitted to an ischemic or a mechanical stress, as defined in the present specification. It will in particular be used separately in the case of pressure and/or volume overload of the right ventricle.

Said product can be in the form of a pharmaceutical composition, which may contain pharmaceutically acceptable excipient.

The invention will be better understood with the following examples. The examples are referring to figures
Figure 1 : cardiac LSD1 activity after 1 month treatment
Figure 2 A: myocardial fibrosis area (left ventricle : LV) after treatment by GSK-LSD1 compared to a losartan treatment (in a model of permanent ischemia)
Figure 2B left ventricular wall thickness after treatment by GSK-LSD1 compared to a losartan treatment
Figure 3A : myocardial fibrosis area (LV) after treatment by GSK-LSD11 in ischemia reperfusion model
Figure 3B : Left ventricular function expressed as shortening fraction (SF) with or without treatment by GSK-LSD1, in ischemia reperfusion model

Pretreatment refers to the time point post-Myocardial infarction and before the start of the GSK LSD1 treatment of mice.

### Example : effect of LSD1 inhibitor on development of cardiac fibrosis after experimental myocardial infarction in mice

The efficacy of an inhibitor of LSD1 (i.e. GSK-LSD1) is tested on the extent of cardiac fibrosis after experimental infarction in mice, with a comparison with losartan treatment.

Six eight-week-old C57BL/6 adult mice are treated with GSK-LSD1, and a control group of six mice are treated with losartan.

Both groups were subjected to experimental infarction.
A control group received no treatment.

Two myocardial infraction models were used, respectively a permanent infarction model and an ischemia -reperfusion model.

The LSD1 inhibitor (1.5 mg / Kg) was administered by intragastric gavage 10 days after experimental infarction and for three weeks. Losartan (5mg/kg) was also administered by oral gavage one week after experimental infarction and for three weeks,

### Experimental sequence:

1: experimental infarctus :
2.: 10 days later: control echo : pretreatment echo:
3: posttreatment echo one month later

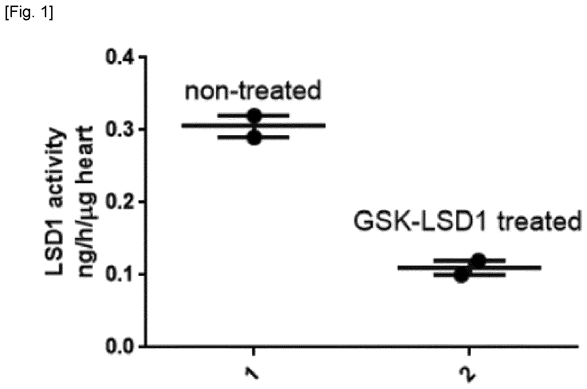

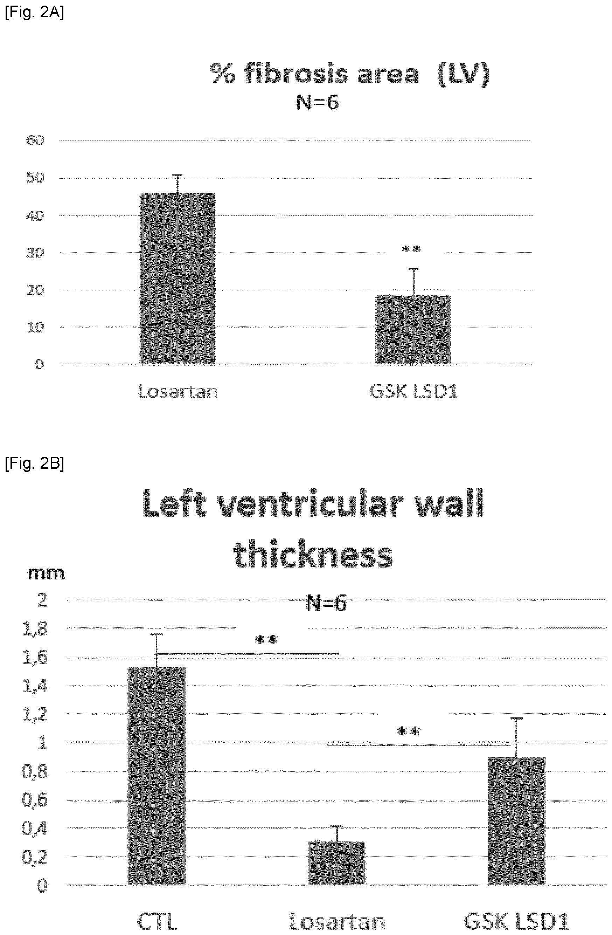

The severity of the infarction was assessed by echocardiography 10 days after surgery. LSD1 activity was measured at the end of treatment (Epigentec kit) in explanted hearts. It is significantly reduced by the GSK-LSD1 (Figure 1)
**1-** Permanent infarction model : ligation of descending anterior left coronary, permanent ligation, with no reperfusion.
   In this first model, the inhibitor GSK-LSD1 significantly reduced fibrosis
   Figure 2 A shows the quantification of the myocardial fibrosis area using Image J) compared to a Losartan (5mg/kg) treatment, losartan being an angiotensin receptor inhibitor, angiotensin being only one of the pro-fibrotic agent in heart failure. The ventricular wall has thickened when compared to the group treated with losartan Student -t-test ** p≤ 0.001 (Figure 2 B).
   Left ventricular function has improved.
**2-** Ischemia -reperfusion model : transient ligation of descending anterior left coronary (30 min); followed by reperfusion

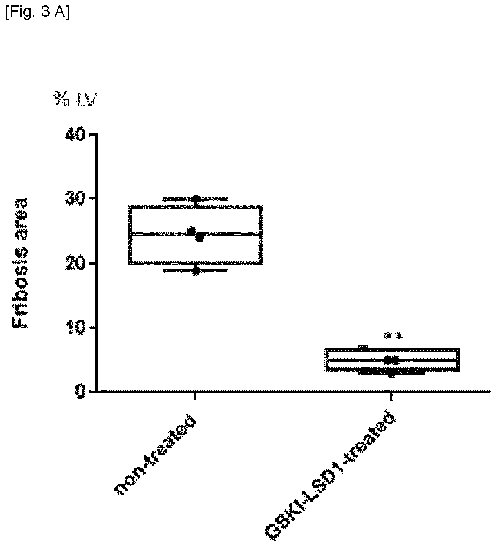

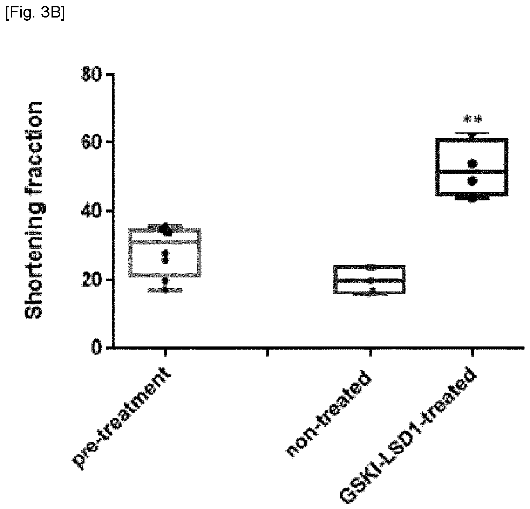

The results were similar in the ischemia / reperfusion model. GSK-LSD1 prevented fibrosis when compared with the non treated group (Figure 3 A). Student -t-test **p≤ 0.001
Figure 3 B shows that GSK-LSD1 fully restored left ventricular function as indicated by an improvement of the shortening fraction, as calculated from the M-mode echocardiography, when compared to the non-treated group and the values obtained before the treatment (pretreatment) (Figure 3 B). Student -t-test **p≤ 0.001

The shortening fraction (SF) is an index of contractile activity : the contraction of the walls of ventricle is responsible of the shortening of the distance between each wall. This is calculated by dividing the distance between the walls at diastole minus the distance between the walls at systole by the distance between the walls at diastole (in %) Shortening fraction x Volume of chamber = ejection fraction (% of the chamber volume emptied at each contraction)

The normal SF in healthy mice is 60%

Post infarct it goes down to 20% ;
Post administration of GSKLSD1, the SF is fully restored back up to 60%

## Claims

1. An LSD1 inhibitor for use in preventing, reducing or stopping a development of a fibrosis of a myocardial tissue, said tissue having been submitted to an ischemic or a mechanical stress.

2. An LSD1 inhibitor for use according to claim 1 in preventing, reducing or stopping a development of a fibrosis of the myocardial tissue further to a myocardial infarction.

3. An LSD1 inhibitor for use according to claim 1 in preventing, reducing or stopping a development of a fibrosis of myocardial tissue of the right ventricle of the heart, following pressure and/or volume overload of the right ventricle.

4. An LSD1 inhibitor for use according to claim 3 in preventing, reducing or stopping a development of a fibrosis of a tissue further to surgery of an individual having a condition selected from tetralogy of Fallot, double outlet right ventricle, transposition of great arteries and Systemic right ventricle.

5. An LSD1 inhibitor for use according to any one of claims 1 to 4, for inhibiting or controlling myocardial localized fibrosis.

6. An LSD1 inhibitor for use according to any one of claims 1 to 5, wherein the LSD1 inhibitor is selected from the group comprising irreversible LSD1 inhibitors and reversible LSD1 inhibitors.

7. An LSD1 inhibitor for use according to any one of claims 1 to 6, wherein the LSD1 inhibitor is selected from GSK-LSD1, GSK-2879552, ORY-1001, SP2509, SP2577, TCP, IMG-7289, INCB059872, ORY-2001, CBB3001, RG6016, INCB059872, IMG-7289, CC-90011 and micro RNA.

8. An LSD1 inhibitor for use according to any one of claims 1 to 7, wherein the LSD1 inhibitor is used as the sole active ingredient.

9. An LSD1 inhibitor for use according to any one of claims 1 to 7, wherein the LSD1 inhibitor is used in association with at least another active ingredient.

10. An LSD1 inhibitor for use according to any one of claims 1 to 9, wherein the LSD1 inhibitor is administered in a composition comprising one or more pharmaceutically acceptable diluents or carriers.

11. An LSD1 inhibitor for use in preventing, reducing or stopping a development of a fibrosis of a tissue according to any one of claims 1, 2 or 5 to 10, wherein the tissue has been subjected to an ischemic stress which is myocardial infarction, and administration of the LSD1 inhibitor begins between 2 to 3 months after said ischemic stress.

12. An LSD1 inhibitor for use according to claim 11, wherein the LSD1 inhibitor is used in association with at least another active ingredient selected from the group comprising beta blocking agents, anticoagulant, ACE (angiotensin-converting enzyme) inhibitors, Angiotensin II receptor blockers, Calcium channel blockers, diuretics, β-adrenergic receptors blockers, pacemaker channels modulators, digoxine (digitalis).

13. An LSD1 inhibitor for use in preventing, reducing or stopping a development of a fibrosis of a tissue according to any one of claims 1 or 3 to 10, wherein the tissue has been subjected to a mechanical stress resulting from a surgical operation and administration of the LSD1 inhibitor begins one week after said mechanical stress.

14. Product containing (i) at least an LSD1 inhibitor and (ii) an active ingredient selected from the group comprising beta blocking agents, anticoagulant, ACE (angiotensin-converting enzyme) inhibitors, Angiotensin II receptor blockers, Calcium channel blockers, diuretics, β-adrenergic receptors blockers, pacemaker channels modulators, digoxin (digitalis) as a combined preparation for simultaneous, separate or sequential use in preventing or reducing a development of a fibrosis of a tissue, said tissue having been submitted to an ischemic stress, as defined in any of the preceding claims.
